(19) Europäisches Patentamt
European Patent Office
Office européen des brevets

(11) Numéro de publication: **0 519 139 B1**

(12) # FASCICULE DE BREVET EUROPEEN

(45) Date de publication du fascicule du brevet: **08.11.95**

(51) Int. Cl.[6]: **C07D 303/16**, C07D 301/32

(21) Numéro de dépôt: **91401562.3**

(22) Date de dépôt: **12.06.91**

(54) **Procédé de purification du (méth)acrylate de glycidyle.**

(43) Date de publication de la demande:
**23.12.92 Bulletin 92/52**

(45) Mention de la délivrance du brevet:
**08.11.95 Bulletin 95/45**

(84) Etats contractants désignés:
**AT BE CH DE DK ES FR GB GR IT LI LU NL SE**

(56) Documents cités:

**CHEMICAL ABSTRACTS, vol. 105, no. 10, 8 Septembre 1986, Columbus, Ohio, US; abstract no. 79495H, page 9 ; & SU-A-1 175 931 (V.A. Stepanova et al.)**

**CHEMIE INGENIEUR TECHNIK vol. 45, no. 14, 1973, WEINHEIM DE pages 942 - 945; B. HEGNER ET AL.: 'Möglichkeiten der Berechnung bei heteroazeotroper Destillation'**

(73) Titulaire: **ELF ATOCHEM S.A.**
**4 & 8, Cours Michelet**
**La Défense 10**
**F-92800 Puteaux (FR)**

(72) Inventeur: **Hess, Raymond**
**21, rue Lepinsek**
**F-57600 Forbach (FR)**
Inventeur: **Lacroix, Christian**
**11, rue de l'Eglise**
**F-57600 Folkling (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 519 139 B1

## Description

La présente invention porte sur un procédé de purification des méthacrylate et acrylate de glycidyle. Ces composés sont des intermédiaires de synthèse intéressants de par leur double liaison de monomère acrylique polymérisable et de par leur fonction époxy.

Les méthacrylate et acrylate de glycidyle (désignés ci-après respectivement par les abréviations MAGLY et AGLY) sont obtenus, d'une manière générale, par les deux étapes réactionnelles suivantes :

(a) la neutralisation des acides respectivement méthacrylique et acrylique par une base, telle qu'un carbonate ou hydrogénocarbonate alcalin anhydre, avec l'épichlorhydrine comme solvant, ce qui conduit au sel d'acide solide, lequel reste en suspension dans l'épichlorhydrine, avec dégagement de dioxyde de carbone et d'eau, selon le schéma réactionnel ci-après (mettant en jeu un carbonate de métal alcalin) :

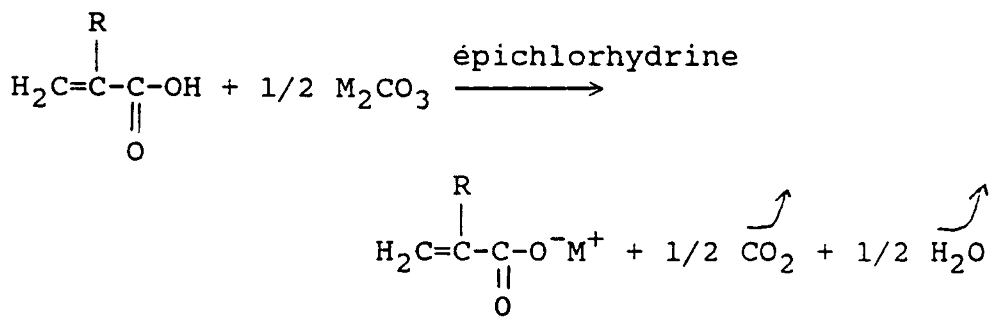


(R = H, $CH_3$) (M = métal alcalin)

On conduit la réaction avec un excès du carbonate par rapport à l'acide de départ, dans un réacteur du type Grignard, agité et chauffé à une température d'environ 90-100 °C. A titre d'exemple, on utilise un mélange réactionnel avec un rapport molaire épichlorhydrine/carbonate/acide (méth)acrylique de l'ordre de 6/0,7/1. L'eau formée lors de la neutralisation est éliminée au fur et à mesure de sa formation par distillation hétéroazéotropique du mélange eau-épichlorhydrine, de manière à éviter les réactions secondaires entre l'eau et l'épichlorhydrine. On condense l'hétéroazéotrope, on le décante à la température ambiante, on soutire la phase aqueuse supérieure, et on recycle l'épichlorhydrine dans le réacteur, pour obtenir un sel d'acide restant en suspension, afin d'éviter l'obtention de mélanges pâteux très chargés en sels d'acides.

(b) la réaction du sel d'acide avec l'épichlorhydrine après introduction, comme catalyseur, d'un sel d'ammonium quaternaire, comme le chlorure de triméthylbenzylammonium, le chlorure de triéthylbenzylammonium, le chlorure de tétraméthylammonium et le bromure de tétraméthylammonium, ou une amine tertiaire, comme la triéthylamine, la tributylamine, la triphénylamine, la diméthylaniline ou la pyridine.

Le schéma réactionnel d'ensemble est le suivant :

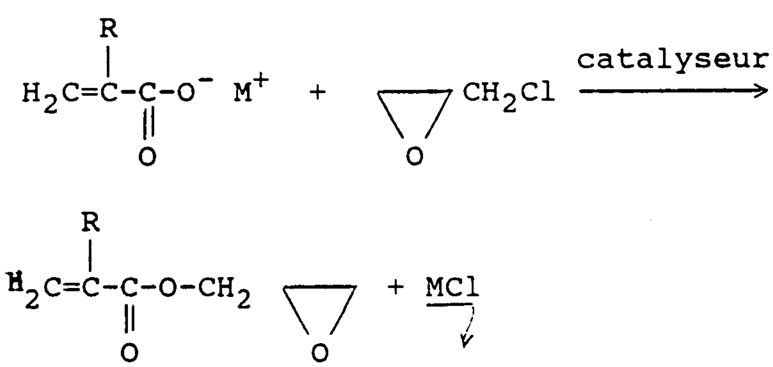


Pour conduire cette étape, on introduit le catalyseur directement dans le mélange obtenu à la première étape, qui comporte le sel d'acide formé, le solvant (qui est dorénavant un réactif), et l'excès de carbonate de départ, la température étant de l'ordre de 95-100 °C. Le sel MCl précipite, et il se forme le

MAGLY ou l'AGLY qui sont liquides. Le mélange réactionnel final brut contient, en suspension, des sels solides, à savoir l'excès de carbonate de sodium de départ et le MCl formé qui sont séparés, par exemple, par filtration. Le mélange réactionnel brut filtré a notamment la composition suivante :

| | |
|---|---|
| Epichlorhydrine .................... | 70-80% en poids |
| MAGLY ou AGLY ...................... | 15-25% " " |
| Impuretés lourdes (ayant un point d'ébullition > celui du (M)AGLY) ... | 2-5% " " |
| Impuretés légères (ayant un point d'ébullition < celui du (M)AGLY) ... | 1-2% " " |

Pour purifier le MAGLY ou l'AGLY, de façon classique, on soumet le mélange réactionnel brut filtré à une distillation fractionnée en discontinu pour séparer les impuretés légères et l'épichlorhydrine (étêtage), puis les impuretés lourdes (équeutage).

Le problème qui se pose alors est celui de la polymérisation du MAGLY ou de l'AGLY, laquelle peut se produire dès que la température atteint au moins 70 °C dans le bouilleur. La polymérisation présente l'inconvénient d'une part, d'encrasser tout l'appareillage, ce qui conduit à l'arrêt de l'installation, d'autre part, de provoquer une perte en produit. Cette polymérisation est liée à la température, à la durée pendant laquelle cette température a été appliquée, ainsi qu'à l'addition ou non d'au moins un inhibiteur de polymérisation. Pour résoudre ce problème, on peut abaisser la température et/ou diminuer le temps de séjour dans le bouilleur. Pendant la distillation, la température augmente et on diminue progressivement la pression pour maintenir la température constante au niveau du bouilleur. Généralement, en fin de distillation de l'épichlorhydrine (étêtage), la pression absolue est de l'ordre de 1,33 x $10^2$-2,66 x $10^3$ Pa (1-20 mmHg) pour ne pas dépasser 100 °C. Comme mentionné dans DE-A-3 126 943 et FR-A-2 286 823.

Par ailleurs, comme indiqué ci-dessus, on introduit, dans le bouilleur et en tête de la colonne à distiller, au moins un inhibiteur de polymérisation, tel que l'hydroquinone, l'éther méthylique de l'hydroquinone ou la phénothiazine, qui sont efficaces en présence d'air, lequel est introduit en continu dans le bouilleur.

Malgré toutes ces précautions, d'une part, on n'empêche pas totalement les problèmes de polymérisation du MAGLY ou de l'AGLY dans le bouilleur, et, d'autre part, la teneur en épichlorhydrine dans le bouilleur après étêtage est toujours supérieure ou égale à 0,2%, c'est-à-dire trop élevée.

Pour distiller le MAGLY ou l'AGLY, en technique habituelle, la pression absolue dans le bouilleur est de l'ordre de 1,33 x $10^2$-6,65 x $10^2$ Pa (1-5 mmHg), comme indiqué dans les Exemples de la demande de brevet allemand DE-A-3 126 943. L'obtention d'un vide poussé est une opération industriellement très contraignante et nécessite l'utilisation d'une installation parfaitement étanche. De même, le vide obtenu dans le bouilleur est également limité par les pertes de charge de la colonne à distiller.

Par ailleurs, lors de l'équeutage du MAGLY ou de l'AGLY, l'épuisement du résidu lourd dans le bouilleur est également limité par les risques de polymérisation dès que la température dans le bouilleur atteint au moins 70 °C environ.

Pour résoudre ces difficultés, il est proposé, conformément au brevet CA-A-986 126, d'introduire un gaz inerte en pied de colonne de distillation de l'épichlorhydrine, de telle sorte que les traces d'épichlorhydrine restantes soient entraînées par le gaz inerte. D'une part, l'introduction d'un tel gaz inerte est incompatible avec la présence d'un inhibiteur de polymérisation, et d'autre part, il sort de l'installation des gaz pollués par des traces d'épichlorhydrine, qui sont très difficiles à purifier. L'épichlorhydrine est un produit toxique, dont les teneurs résiduelles dans le MAGLY ou l'AGLY devraient, de préférence, ne pas dépasser 100 ppm. Ainsi, le procédé selon le brevet CA-A-986 126 permettrait de résoudre le problème de l'épichlorhydrine résiduelle - ce, malgré d'autres difficultés, à savoir installations compliquées ou polluantes - mais, en aucun cas, il ne résout le problème lié à la polymérisation du MAGLY ou de l'AGLY.

La Société déposante a donc développé un nouveau procédé de purification du MAGLY ou de l'AGLY faisant intervenir des distillations hétéroazéotropiques de l'épichlorhydrine purs du MAGLY ou de l'AGLY, qui affranchissent des difficultés précédemment décrites.

La présente invention a donc pour objet un procédé de purification par distillation du (méth)acrylate de glycidyle contenant des impuretés légères et des impuretés lourdes, caractérisé par le fait que :

- dans une première étape, on conduit une distillation du (méth)acrylate de glycidyle à purifier en présence d'un premier solvant capable de former un hétéroazéotrope à bas point d'ébullition avec les impuretés légères et l'épichlorhydrine, de façon à obtenir une fraction de tête qui est constituée par

un hétéroazéotrope solvant-produits légers ; et

- dans une deuxième étape, on soumet le (méth)acrylate de glycidyle ainsi débarrassé des produits légers à une distillation en présence d'un second solvant capable de former un hétéroazéotrope à bas point d'ébullition avec le (méth)acrylate de glycidyle, de façon à obtenir une fraction de tête constituée par le (méth)acrylate de glycidyle pur recherché, ainsi débarrassé des impuretés lourdes,

lesdits solvants étant, au cours de ces deux distillations, présents dans toute la zone de distillation, y compris le bouilleur.

Conformément à un premier mode de réalisation, le procédé est conduit en discontinu en éliminant, en première étape, la fraction de tête constituée par l'hétéroazéotrope premier solvant-produits légers, puis en recueillant le (méth)acrylate de glycidyle en seconde étape.

Conformément à un second mode de réalisation, le procédé est conduit en continu dans deux colonnes à distiller distinctes.

De façon préférée, le solvant capable de former un azéotrope est l'eau à chacune des distillations.

Dans les deux modes de réalisation (continu ou discontinu), on peut conduire une étape préliminaire d'élimination, par distillation sous pression réduite, d'une partie prépondérante de l'épichlorhydrine,

- à une température suffisamment faible pour qu'aucune polymérisation du (méth)acrylate de glycidyle n'ait lieu, par exemple ne dépassant pas 70 °C environ, ou bien
- suivant une voie préférée, de façon continue, en choisissant un bouilleur à court temps de séjour, tel qu'un évaporateur à film mince, un évaporateur à film raclé, ou un évaporateur à film tombant, pour lesquels les temps de séjour sont inférieurs à une minute.

Conformément à cette seconde voie, on peut opérer à des niveaux de température d'au moins 80 °C environ, et pouvant aller jusqu'à 130 °C environ.

Lors des étapes d'étêtage et d'équeutage, réalisées en présence d'eau comme solvant, la polymérisation du (méth)acrylate de glycidyle est évitée et le résidu lourd parfaitement épuisé, en opérant sous une pression absolue comprise entre environ $2,66 \times 10^3$ et $1,01 \times 10^5$ Pa (20 et 760 mmHg). préférentiellement entre $1,33 \times 10^4$ et $3,99 \times 10^4$ Pa (100 et 300 mmHg), qui permet de fixer les températures entre 40 et 85 °C.

En outre, il est souhaitable qu'à chacune des étapes de distillation (étape préliminaire et première et seconde étapes), on introduise au moins un inhibiteur de polymérisation, par exemple, du type mentionné dans le préambule de cette description, en quantité équivalente à 10-1000 ppm par rapport au (méth)-acrylate de glycidyle, ainsi qu'un flux d'air compris entre 0,1-10 normaux litres par kilogramme de (méth)-acrylate de glycidyle distillé.

Enfin, on peut vaporiser sous un vide poussé de $1,33 \times 10^3$ à $2,66 \times 10^3$ Pa (10 à 20 mmHg) les traces d'eau contenues dans le (méth)acrylate de glycidyle obtenu.

L'invention sera encore illustrée par la description suivante, faite en référence avec la figure unique du dessin annexé, représentant le diagramme de fonctionnement du procédé. Tous les pourcentages sont donnés en poids sauf indication contraire.

Dans un réacteur agité de type Grignard 1, on conduit la réaction classique de synthèse du (méth)-acrylate de glycidyle, en deux étapes, tel qu'indiqué dans le préambule de la description. Lors de la première étape, comme indiqué, l'eau formée est éliminée par distillation azéotropique du mélange eau-épichlorhydrine. L'azéotrope est donc évacué en tête par le conduit 2, il est condensé, à la température ambiante, dans le condenseur 3, puis décanté dans le décanteur 4, à la partie supérieure duquel le dioxyde de carbone est évacué en 5, et dans lequel l'eau et l'épichlorhydrine se séparent en une phase respectivement supérieure et inférieure, l'eau étant évacuée en 6, et l'épichlorhydrine étant recyclée en 7 dans le réacteur 1.

En fin de seconde étape, le mélange réactionnel brut est soutiré en 8, et transféré vers un dispositif de séparation 9, par exemple, un filtre, duquel les sels solides (excès du carbonate de sodium de départ et MCl formé) sont retirés en 10, le mélange réactionnel brut 11, ainsi débarrassé des impuretés solides, étant alors transféré vers un bac tampon 12, dans lequel est introduit, en 13, un stabilisant (inhibiteur de polymérisation), par exemple, d'hydroquinone.

Le mélange brut ainsi stabilisé alimente en continu une colonne à distiller 16, équipée d'un bouilleur à faible temps de séjour, tel qu'un évaporateur à film mince 16<u>a</u>. Dans le bouilleur, la température est fixée à 90 °C-130 °C, de préférence. La distillation est effectuée sous un vide (symbolisé en 17) de l'ordre de $1,33 \times 10^3$ - $3,99 \times 10^3$ Pa absolus (10-30 mmHg absolus), notamment d'environ $2,66 \times 10^3$ Pa absolus (20 mmHg absolus). La colonne à garnissage 16 permet de séparer en tête 16<u>b</u> l'épichlorhydrine suivant le conduit 18, laquelle est condensée en 19 puis évacuée en 20, une partie étant recyclée en 21 dans la colonne 16. La température de tête est de l'ordre de 20 à 40 °C. Pour ces conditions de température de bouilleur à la pression de service, la teneur en épichlorhydrine résiduelle est de l'ordre de 0,2 à 2%. Le

temps de séjour est de quelques secondes, ce qui diminue considérablement tout risque de polymérisation du MAGLY ou de l'AGLY dans le bouilleur. La colonne 16 et le bouilleur sont par ailleurs protégés par l'introduction en continu 22 d'inhibiteur de polymérisation en tête de colonne, et par l'injection d'air 23 au niveau du circuit du bouilleur 16a.

Le courant 24 contenant 0,2 à 2% en poids d'épichlorhydrine résiduelle, qui est soutiré de la colonne 16, alimente une colonne 25 de distillation hétéroazéotropique épichlorhydrine-eau. Cette colonne 25 peut être équipée d'un bouilleur tubulaire, auquel cas les temps de séjour sont de l'ordre de 1-2 heures, ou d'un évaporateur à film (film mince ou film raclé), auquel cas les temps de séjour peuvent être de l'ordre de quelques secondes. Dans cette distillation azéotropique, le temps de séjour n'est pas critique.

A la colonne 25, sont associés un circuit de vaporisation 25a et un circuit de tête 25b qui comprend un conduit 26 d'extraction de l'azéotrope épichlorhydrine-eau, un condenseur 27, un décanteur 28 et un conduit 29 de recyclage d'eau à la colonne 25, Celle-ci fonctionne sous un vide (symbolisé en 30) de 2,66 x $10^3$ à 1,01 x $10^5$ Pa absolus, (20 à 760 mmHg absolus), de préférence de 1,33 x $10^4$ à 3,99 x $10^4$ Pa absolus (100 à 300 mmHg absolus), ce qui fixe la température du bouilleur entre 40 et 85°C. La température du bouilleur est en effet fixée à la température d'ébullition de l'azéotrope à la pression de service. L'eau de formation de l'azéotrope est introduite en 31 dans le décanteur 28.

L'introduction d'eau permet de diminuer fortement les niveaux thermiques dans la colonne 25, et surtout dans le bouilleur associé, empêchant ainsi toute polymérisation du MAGLY ou de l'AGLY.

Du reste, par précaution, un courant 32 de stabilisant est introduit à la partie supérieure de la colonne 25 et un flux d'air 33, au niveau du circuit de vaporisation 25a en pied de colonne.

On obtient donc en tête de la colonne 25 un mélange azéotropique épichlorhydrine-eau (environ 75% d'épichlorhydrine et 25% d'eau) qui, après condensation en 27, est décanté. L'eau est totalement recyclée en 29 sous forme de reflux, et l'épichlorhydrine est soutirée en 34, et, le cas échéant, recyclée dans le conduit 14 d'alimentation de la colonne 16.

En pied, on obtient un mélange brut contenant de l'eau, qui alimente un décanteur 35, dans lequel l'eau se sépare en phase supérieure. Cette eau est recyclée en 36 à la partie inférieure de la colonne 25, et le mélange brut étêté résultant qui est soutiré du décanteur 35, et qui contient moins de 100 ppm d'épichlorhydrine alimente en continu, par le circuit 37, une colonne 38.

La colonne 38 est une colonne de distillation du MAGLY ou de l'AGLY sous forme d'un hétéroazéotrope MAGLY ou AGLY-eau. Cette colonne 38 fonctionne dans des conditions de pression et de température de bouilleur identiques ou voisines de celles de la colonne 25.

Au bouilleur de la colonne 38, est associé, de la même façon qu'à la colonne 25, un décanteur 39 et un conduit 40 de recyclage de l'eau.

Le circuit de tête 38b de la colonne 38 est strictement identique au circuit 25b de la colonne 25. Il comporte une canalisation 41 d'extraction de l'hétéroazéotrope MAGLY ou AGLY-eau - lequel contient environ 90% d'eau et 10% de MAGLY ou d'AGLY -, un condenseur 42, un décanteur 43, et un conduit 44 de recyclage de l'eau à la partie supérieure de la colonne 38. La mise sous vide est symbolisée en 45, et l'introduction d'eau, en 46. De la même façon que précédemment, un courant de stabilisant est introduit en 47 à la partie supérieure de la colonne 38, et une injection d'air 48 est introduite au niveau du circuit de vaporisation 38a.

En tête de la colonne 38, après condensation, décantation et reflux total de l'eau, on soutire en 49 du MAGLY ou de l'AGLY qui contient des traces d'eau à hauteur de 2%. On sèche ce MAGLY ou cet AGLY sous un vide poussé, ce qui permet de recueillir en 50 le MAGLY ou l'AGLY à 0,1% d'eau environ, l'eau résultante étant recyclée dans le décanteur 43 par le conduit 51.

En pied de colonne 38, on soutire en 52 un résidu lourd contenant moins de 10% de MAGLY ou d'AGLY.

TABLEAU

| Comparaison des températures d'ébullition de l'épichlorhydrine, du MAGLY, des hétéroazéotropes épichlorhydrine-eau et MAGLY-eau, du résidu lourd à 10% de MAGLY et du résidu lourd-eau à 10% de MAGLY. | | | | | | | |
|---|---|---|---|---|---|---|---|
| Pression | | Températures d'ébullition (° C) | | | | | |
| en Pa | (mmHg absolu) | Epichlorhydrine | Epichlorhydrine-eau | MAGLY | Résidu lourd à 10% de MAGLY | Résidu lourdeau | MAGLY-eau |
| $6.65 \times 10^2$ | (5) | - | - | 62 | 120 ° C | - | - |
| $2.66 \times 10^3$ | (20) | 28 | 10 | 90 | 140 ° C | 26 | 20 |
| $1.33 \times 10^4$ | (100) | 62 | 38 | 126 | - | 52 | 46 |
| $1.99 \times 10^4$ | (150) | 72 | 46 | - | - | 62 | 56 |
| $2.66 \times 10^4$ | (200) | - | 54 | - | - | 70 | 64 |
| $3.99 \times 10^4$ | (300) | 90 | 64 | 155 | - | 80 | 74 |

EXEMPLE 1 (COMPARATIF) : Distillation frationnée en discontinu

L'alimentation de départ est constituée par 616 g d'un mélange brut tel que celui soutiré en 11 sur la Figure ayant la composition suivante :

| | |
|---|---|
| Epichlorhydrine | 76% |
| MAGLY | 20,9% |
| Lourds | 3% |
| Impuretés totales | 1% |

On conduit un étêtage sous une pression absolue de 1,06 x $10^4$ Pa (80 mmHg) initialement et 5,32 x $10^2$ Pa (4 mmHg) en fin d'opération, la température du bouilleur étant d'environ 80 °C.

On conduit ensuite une distillation du MAGLY sous une pression absolue de 3,99 x $10^2$-5,32 x $10^2$ Pa (3-4 mmHg), pour une température du bouilleur inférieure ou égale à 100 °C.

Les résultats de l'analyse du MAGLY distillé (90 g) sont les suivants :

| | |
|---|---|
| Epichlorhydrine | 0,2% |
| MAGLY | 98,4% |
| Lourds | 0,5% |
| Autres | 0,9%. |

Les résultats de l'analyse du résidu (26 g) sont les suivants :

| | |
|---|---|
| MAGLY | 28% |
| Lourds | 70% |

On observe la présence de polymères solides du MAGLY dès que la température du bouilleur atteint 100 °C ; une forte teneur en épichlorhydrine dans le MAGLY (de l'ordre de 2000 ppm) et une teneur importante en MAGLY dans le résidu.

EXEMPLE 2 : Distillation en continu en trois étapes (dont deux distillations azéotropiques)

On utilise un appareillage du type de celui qui vient d'être décrit en référence à la Figure unique.

Le débit d'alimentation du MAGLY brut est de 460 g/h. Ce MAGLY a la composition suivante :

| | |
|---|---|
| Epichlorhydrine | 79,5% |
| MAGLY | 17,3% |
| Lourds | 3,2% |

On soumet la colonne 16 aux conditions de fonctionnement suivantes :

Pression : 2,66 x $10^3$ Pa absolus (20 mmHg absolus)

Température de tête : 30 °C

Température du bouilleur : 110 °C.

Le débit de la fraction de tête, laquelle est à 99,8% d'épichlorhydrine, est de 359/h.

On soumet la colonne 25, en présence d'eau, aux conditions de fonctionnement suivantes :

Pression : 1,99 x $10^4$ Pa absolus (150 mmHg absolus)

Température de tête : 50 °C

Température du bouilleur : 60 °C

Le débit de la fraction de tête, après décantation, est de 19,2 g/h. Cette fraction a la composition suivante :

| | |
|---|---|
| Epichlorhydrine | 14% |
| MAGLY | 83% |
| Lourds | 3% |

On soumet la colonne 38, en présence d'eau, aux conditions de fonctionnement suivantes :
Pression : 1,99 x $10^4$ Pa absolus (150 mmHg absolus)
Température de tête : 52 °C
Température du bouilleur : 62 °C.

Les débits de la fraction de tête distillée après séchage et du résidu sont respectivement de 61,4 g/h et de 19,3 g/h.

Cette fraction de tête distillée après séchage a la composition suivante :

| | |
|---|---|
| Epichlorhydrine | <100 ppm |
| MAGLY | >99,0% |
| Lourds | <1% |

Le résidu a la composition suivante :

| | |
|---|---|
| Epichlorhydrine | 0% |
| MAGLY | 10% |
| Lourds | 90% |

On n'a observé aucune trace de polymères solides dans l'installation.

**Revendications**

**1.** Procédé de purification par distillation du (méth)acrylate de glycidyle contenant des impuretés légères et des impuretés lourdes, caractérisé par le fait que :
- dans une première étape, on conduit une distillation du (méth)acrylate de glycidyle à purifier en présence d'un premier solvant capable de former un hétéroazéotrope à bas point d'ébullition avec les impuretés légères et l'épichlorhydrine, de façon à obtenir une fraction de tête qui est constituée par un hétéroazéotrope solvant-produits légers ; et
- dans une deuxième étape, on soumet le (méth)acrylate de glycidyle ainsi débarrassé des produits légers à une distillation en présence d'un second solvant capable de former un hétéroazéotrope à bas point d'ébullition avec le (méth)acrylate de glycidyle, de façon à obtenir une fraction de tête constituée par le (méth)acrylate de glycidyle pur recherché, ainsi débarrassé des impuretés lourdes,

lesdits solvants étant, au cours de ces deux distillations, présents dans toute la zone de distillation, y compris le bouilleur.

**2.** Procédé selon la revendication 1, caractérisé par le fait qu'il est conduit en discontinu en éliminant, en première étape, la fraction de tête constituée par l'hétéroazéotrope premier solvant-produits légers, puis, en recueillant le (méth)acrylate de glycidyle en seconde étape.

**3.** Procédé selon la revendication 1, caractérisé par le fait qu'il est conduit en continu dans deux colonnes à distiller distinctes.

**4.** Procédé selon l'une des revendications 1 à 3, caractérisé par le fait que le solvant capable de former un azéotrope est l'eau à chacune des distillations.

**5.** Procédé selon l'une des revendications 1 à 4, caractérisé par le fait qu'on conduit une étape préliminaire d'élimination, par distillation sous pression réduite, d'une partie prépondérante de l'épichlorhydrine, à une température suffisamment faible pour qu'aucune polymérisation du (méth)acrylate de glycidyle n'ait lieu.

6. Procédé selon l'une des revendications 1 à 4, caractérisé par le fait qu'on conduit une étape préliminaire d'élimination, par distillation sous pression réduite, d'une partie prépondérante de l'épichlorhydrine, de façon continue, en choisissant un bouilleur à court temps de séjour.

7. Procédé selon la revendication 6, caractérisé par le fait qu'on opère à des niveaux de température d'au moins 80 °C, et pouvant aller jusqu'à 130 °C.

8. Procédé selon l'une des revendications 1 à 7, caractérisé par le fait qu'à la première et à la seconde étape, on opère sous une pression absolue comprise entre $2,66 \times 10^3$ et $1,01 \times 10^5$ Pa (20 et 760 mmHg) et préférentiellement entre $1,33 \times 10^4$ et $3,99 \times 10^4$ Pa, ce qui permet de fixer les températures entre 40 et 85 °C.

9. Procédé selon l'une des revendications 1 à 8, caractérisé par le fait qu'à chacune des étapes de distillation, on introduit au moins un inhibiteur de polymérisation en quantité équivalente à 10 - 1000 ppm par rapport au (méth)acrylate de glycidyle, et un flux d'air compris entre 0,1 - 10 normaux litres par kilogramme de (méth)acrylate de glycidyle distillé.

10. Procédé selon l'une des revendications 1 à 9, caractérisé par le fait qu'on vaporise sous vide poussé les traces d'eau contenues dans le (méth)acrylate de glycidyle obtenu.

## Claims

1. Process for the purification by distillation of glycidyl (meth)acrylate containing light impurities and heavy impurities, characterized in that:
   - in a first stage, a distillation of the glycidyl (meth)acrylate to be purified is conducted in the presence of a first solvent capable of forming a heteroazeotrope of low boiling point with the light impurities and epichlorohydrin, so as to obtain a head fraction which consists of a solvent-light products heteroazeotrope; and
   - in a second stage, the glycidyl (meth)acrylate thus freed from the light products is subjected to a distillation in the presence of a second solvent capable of forming a heteroazeotrope of low boiling point with glycidyl (meth)acrylate, so as to obtain a head fraction consisting of the required pure glycidyl (meth)acrylate, thus freed from the heavy impurities,

   the said solvents being present, during these two distillations, throughout the distillation zone, including the boiler.

2. Process according to Claim 1, characterized in that it is conducted noncontinuously by removing, in a first stage, the head fraction consisting of the first solvent-light products heteroazeotrope, and by then collecting glycidyl (meth)acrylate in a second stage.

3. Process according to Claim 1, characterized in that it is conducted continuously in two separate distillation columns.

4. Process according to one of Claims 1 to 3, characterized in that the solvent capable of forming an azeotrope is water in each of the distillations.

5. Process according to one of Claims 1 to 4, characterized in that a preliminary stage is conducted to remove, by distillation under reduced pressure, a predominant part of the epichlorohydrin at a sufficiently low temperature for no polymerization of the glycidyl (meth)acrylate to take place.

6. Process according to one of Claims 1 to 4, characterized in that a preliminary stage is conducted to remove a preponderant part of the epichlorohydrin continuously by distillation under reduced pressure, a boiler with a short residence time being chosen.

7. Process according to Claim 6, characterized in that the operation is carried out at temperature levels of at least 80 °C and capable of going up to 130 °C.

8. Process according to one of Claims 1 to 7, characterized in that, in the first and second stage, the operation is carried out at an absolute pressure of between $2.66 \times 10^3$ and $1.01 \times 10^5$ Pa (20 and 760

mm Hg) and preferably between 1.33 × $10^4$ and 3.99 × $10^4$ Pa, which allows the temperatures to be set between 40 and 85 °C.

9. Process according to one of Claims 1 to 8, characterized in that at least one polymerization inhibitor, in a quantity equivalent to 10 - 1000 ppm relative to glycidyl (meth)acrylate, and an air flow of between 0.1 - 10 standard litres per kilogram of glycidyl (meth)acrylate distilled are introduced at each of the distillation stages.

10. Process according to one of Claims 1 to 9, characterized in that the traces of water present in the glycidyl (meth)acrylate obtained are vaporized in a high vacuum.

**Patentansprüche**

1. Verfahren zur destillativen Reinigung von Glycidylmethacrylat und Glycidylacrylat, die niedrigersiedende und höhersiedende Verunreinigungen enthalten, dadurch **gekennzeichnet,** daß
   - in einem ersten Schritt eine Destillation des zu reinigenden Glycidylacrylats bzw. Glycidylmethacrylats in Gegenwart eines ersten Lösungsmittels durchgeführt wird, das mit den niedrigersiedenden Verunreinigungen und Epichlorhydrin ein Heteroazeotrop mit niedrigem Siedepunkt zu bilden vermag, um eine Kopffraktion zu erhalten, die aus einem Heteroazeotrop aus Lösungsmittel und niedrigersiedenden Produkten besteht, und
   - in einem zweiten Schritt das so von den niedrigersiedenden Produkten befreite Glycidylacrylat bzw. Glycidylmethacrylat einer Destillation in Gegenwart eines zweiten Lösungsmittels unterzogen wird, das mit dem Glycidylacrylat bzw. Glycidylmethacrylat ein Heteroazeotrop mit niedrigem Siedepunkt zu bilden vermag, um eine Kopffraktion zu erhalten, die aus dem gewünschten reinen Glycidylacrylat bzw. Glycidylmethacrylat besteht, das so von den höhersiedenden Verunreinigungen befreit wird,

   wobei die obengenannten Lösungsmittel während dieser beiden Destillationen in der gesamten Destillationszone einschließlich des Verdampfers vorhanden sind.

2. Verfahren nach Anspruch 1,
   dadurch gekennzeichnet, daß
   es diskontinuierlich geführt wird, wobei im ersten Schritt die Kopffraktion entfernt wird, die aus dem ersten Heteroazeotrop aus Lösungsmittel und niedrigersiedenden Produkten besteht, und anschließend im zweiten Schritt das Glycidylacrylat bzw. Glycidylmethacrylat gewonnen wird.

3. Verfahren nach Anspruch 1,
   dadurch gekennzeichnet, daß
   es kontinuierlich in zwei verschiedenen Destillationskolonnen geführt wird.

4. Verfahren nach einem der Ansprüche 1 bis 3,
   dadurch gekennzeichnet, daß
   bei jeder Destillation Wasser das Lösungsmittel ist, das das Azeotrop zu bilden vermag.

5. Verfahren nach einem der Ansprüche 1 bis 4,
   dadurch gekennzeichnet, daß
   ein vorbereitender Schritt durchgeführt wird, bei dem der überwiegende Teil des Epichlorhydrins bei einer Temperatur, die so niedrig gewählt ist, daß keine Polymerisation des Glycidylacrylats bzw. Glycidylmethacrylats stattfindet, durch Destillation unter vermindertem Druck entfernt wird.

6. Verfahren nach einem der Ansprüche 1 bis 4,
   dadurch gekennzeichnet, daß
   ein vorbereitender Schritt durchgeführt wird, bei dem der überwiegende Teil des Epichlorhydrins durch Destillation unter vermindertem Druck kontinuierlich entfernt wird, indem ein Verdampfer mit kurzer Verweilzeit ausgewählt wird.

7. Verfahren nach Anspruch 6,
   dadurch gekennzeichnet, daß
   bei Temperaturen von mindestens 80 °C gearbeitet wird, wobei Temperaturen von bis zu 130 °C

möglich sind.

8. Verfahren nach einem der Ansprüche 1 bis 7, dadurch gekennzeichnet, daß im ersten und zweiten Schritt bei einem absoluten Druck von $2{,}66 \cdot 10^3$ und $1{,}01 \cdot 10^5$ Pa (20 - 760 mm Hg), vorzugsweise $1{,}33 \cdot 10^4$ bis $3{,}99 \cdot 10^4$ Pa, gearbeitet wird, was es ermöglicht, die Temperaturen bei 40 bis 85 °C zu halten.

9. Verfahren nach einem der Ansprüche 1 bis 8, dadurch gekennzeichnet, daß bei jedem Destillationsschritt mindestens ein Polymerisationsinhibitor in einer Menge von 10 bis 1000 ppm, bezogen auf das Glycidylacrylat bzw. Glycidylmethacrylat, zugesetzt und ein Strom von 0,1 bis 10 l Luft im Normzustand (Nl) Pro Kilogramm destilliertes Glycidylacrylat bzw. Glycidylmethacrylat zugeführt wird.

10. Verfahren nach einem der Ansprüche 1 bis 9, dadurch gekennzeichnet, daß die in dem erhaltenen Glycidylacrylat bzw. Glycidylmethacrylat enthaltenen Wasserspuren im Hochvakuum verdampft werden.

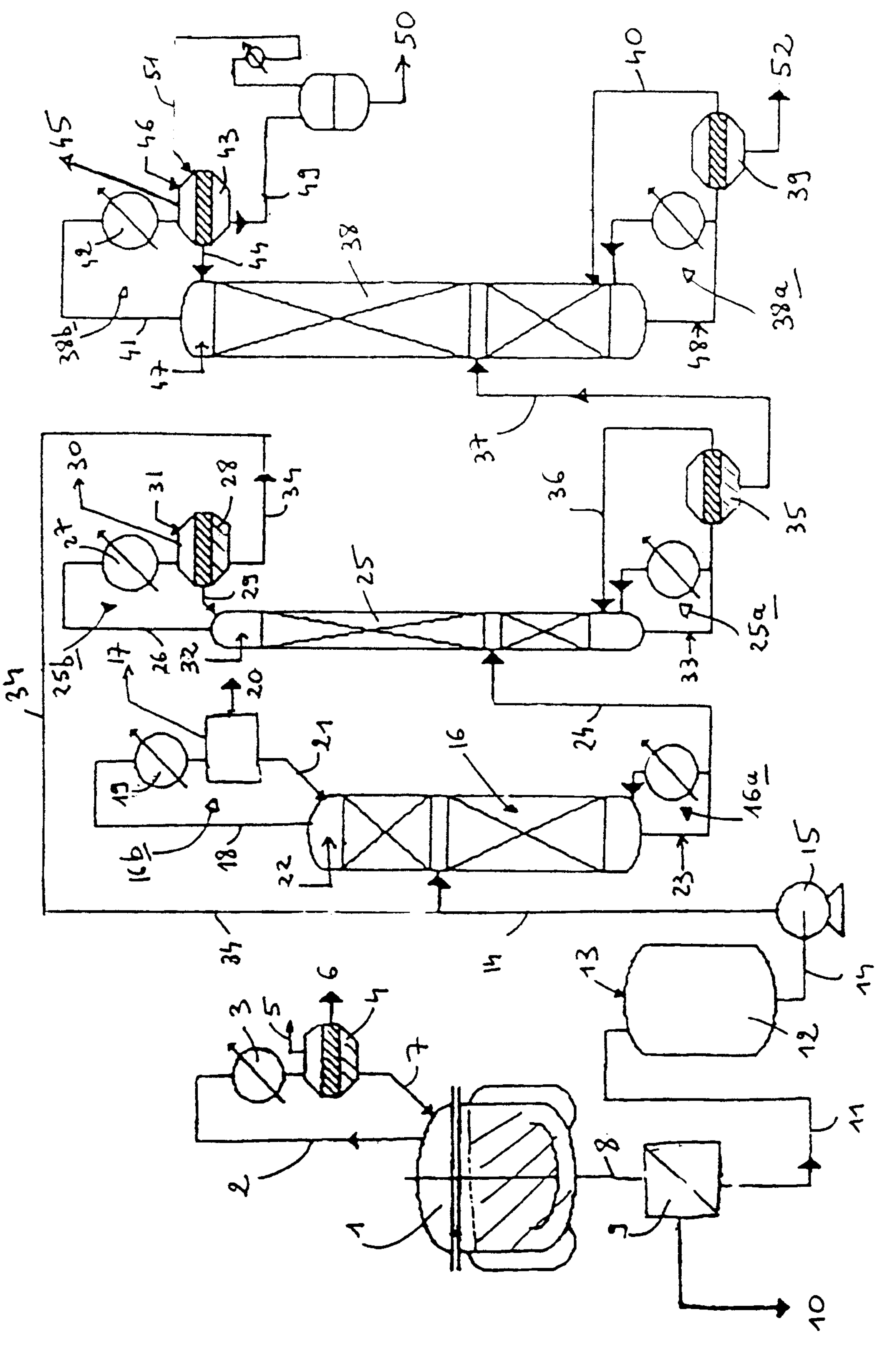

Figure unique